# EUROPÄISCHE PATENTSCHRIFT

(11) **EP 0 397 043 B1**
(45) Veröffentlichungstag und Bekanntmachung des Hinweises auf die Patenterteilung: **23.02.1994**
(21) Anmeldenummer: 90108431.9
(22) Anmeldetag: 04.05.1990
(51) Int. Cl.: A61B 17/36, A61F 7/00, F25B 21/04

(54) **Gerät zur Erzeugung von Kälte und Wärme**
Apparatus for generating cold and heat
Dispositif pour la génération du froid et de la chaleur

(30) Priorität: 09.05.1989 DE 8905769 U
(43) Veröffentlichungstag der Anmeldung: 14.11.1990
(73) Patentinhaber: Schulte, Franz-Josef, Dipl.-Ing, D-59939 Olsberg (DE)
(72) Erfinder: Schulte, Franz-Josef, Dipl.-Ing, D-59939 Olsberg (DE)
(74) Vertreter: Dörner, Lothar, Dipl.-Ing.

(56) Entgegenhaltungen:
- EP-A- 0 239 048
- WO-A-81/03608
- DE-A- 3 327 082
- US-A- 4 090 517

## Beschreibung

Die Erfindung betrifft ein elektronisch gesteuertes Gerät zur Erzeugung von Kälte und Wärme, bei dem an beiden Außenflächen eines von einem Strom steuerbarer Größe durchflossenen Peltier-Elements Platten aus leitendem Material anliegen.

Es ist bekannt, Warzen dadurch zu entfernen, daß man sie täglich mehrfach hintereinander im Wechsel unter einen heißen Wasserstrahl hält und anschließend sofort durch Eiswürfel kühlt. Es wird weiterhin vermutet, daß auch chronische Hautexeme - unbekannter Herkunft - auf solche im schnellen Wechsel erfolgende Warm-Kalt-Applizierungen positiv reagieren. Das angewendete Verfahren - heißer Wasserstrahl, Eiswürfel - ist jedoch verbesserungsfähig.

Bei einem bekannten Gerät der eingangs genannten Art (WO-A-81/03608), das als "thermoelektrisches diagnostisches Instrument" bezeichnet ist, ist im Innern ein Peltier-Modul angeordnet. Das Peltier-Modul weist Platten auf, von denen die eine erwärmt und die andere gekühlt ist. Mit den Platten sind Thermo-Leiter in Form dünner Streifen aus Kupfer verbunden. Die Streifen sind parallel zueinander in eine Kappe eingebettet. Am äußeren Ende der Kappe sind die Streifen nach außen abgewinkelt, sodaß sie Kontaktelemente bilden, die auf gegenüberliegenden Seiten aus der Kappe austreten Bei dem bekannten Gerät, welches vorzugsweise zur Diagnose von Pulpa im zahnärztlichen Bereich eingesetzt wird, kann durch Drehen der Kappe einmal das kalte Element an einen Zahn angelegt werden, zum anderen das warme Element.

Der Erfindung liegt die Aufgabe zugrunde, ein Gerät der eingangs genannten Art so auszubilden, daß die bei einem Peltier-Element ohnehin vorhandene Temperaturdifferenz vergrößert wird, dabei die Anwendungsmöglichkeiten erweitert werden. Gemäß der Erfindung wird diese Aufgabe dadurch gelost, daß jede Platte mit einer auf- und abschiebbaren Behandlungsspitze versehen und die an der warmen Seite des Peltier-Elements anliegende Platte zusätzlich beheizbar ist.

Ein Peltier-Element macht von folgendem thermoelektrischen Effekt Gebrauch: Wird an einen metallischen Leiter A zu beiden Seiten ein metallischer Leiter B angelötet und durch BAB ein Gleichstrom geschickt, so erwärmt sich die eine Lötstelle, während sich die zweite abkühlt. Die Wärmemenge, nämlich die Peltier-Wärme, die an der Verbindungsstelle von A und B (zusätzlich zur Jouleschen Wärme) erzeugt oder aufgenommen wird, ist der Stromstärke des von A nach B fließenden Stromes und der Zeit proportional. Bei der Erfindung ist die an der warmen Seite des Peltier-Elements anliegende Platte zusätzlich erwärmt. Dies hat zur Folge, daß die Temperaturdifferenz noch vergrößert werden kann.

Mit der Erfindung ist es nicht nur möglich, die angesprochenen Kalt-Warm-Reize in der Medizin zur Beseitigung von Krankheiten im schnellen Wechsel einfach aufzubringen, wobei die umliegenden gesunden Partien geschont werden; sondern auch in der Technik, zum Beispiel bei der Materialprüfung, ein Temperaturgefälle zu erzeugen.

Die Behandlungsspitzen können flächenartig gestaltet sein, um z.B. ganze Hautpartien zu behandeln.

Die an der warmen Seite des Peltier-Elements anliegende Platte ist zusätzlich erwärmbar. Dies hat zur Folge, daß die Temperaturdifferenz noch vergrößert werden kann.

Bei Peltier- Elementen wird die kalte Seite nach kurzer Zeit über das Halbleitermaterial aufgeheizt. Aus diesem Grund muß die warme Seite ausreichend gekühlt werden. Üblicherweise geschieht dies mittels Luftkühlung - Lüfter; Gebläse - oder Wasserkühlung der warmen Seite. In Ausgestaltung der Erfindung ist auf der warmen Seite des Peltier-Elements ein Lüfter vorgesehen. In Kauf genommen wird dabei das träge Ansprechverhalten bei der Luftkühlung und der störende Wasseranschluß bei der Wasserkühlung.

Zur Verbesserung der Regelung - exaktes Einhalten der vorgewählten Temperatur auf der kalten Seite - wird in einer anderen Weiterbildung der Erfindung folgende Anordnung gewählt: Zwei Peltier-Elemente sind so kaskadiert, daß an der warmen Seite des ersten Elements die kalte Seite des zweiten Elements direkt angrenzt. Die warme Seite des zweiten Elements wird bei Bedarf mit einem Lüfter konstant oder ebenfalls gesteuert gekühlt. Da die Peltier-Elemente durch Stromsteuerung verhältnismäßig schnell und sehr exakt gesteuert werden können, ergibt sich bei der genannten Weiterbildung eine erheblich größere Temperaturkonstanz der kalten Seite des ersten Peltier-Elements auch bei Wärmezufuhr an dieser Seite.

Weitere Ausgestaltungen der Erfindung sind in weiteren Unteransprüchen angegeben.

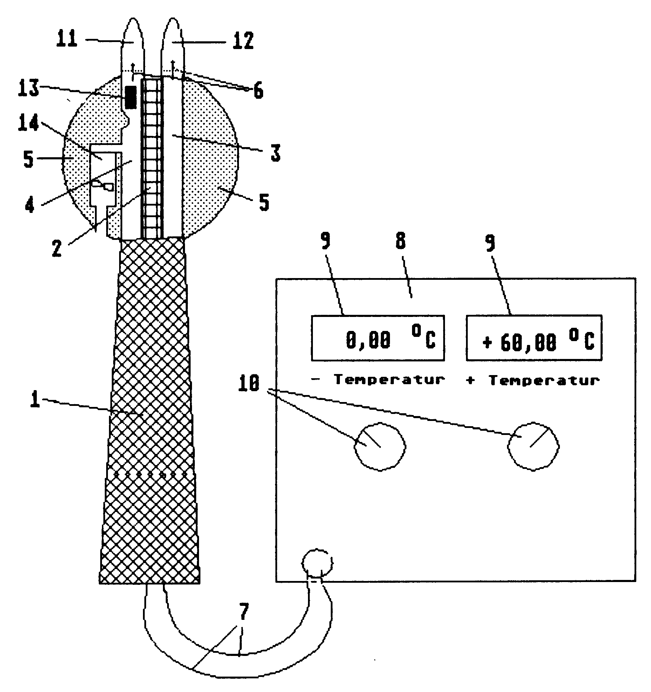

Ein Ausführungsbeispiel der Erfindung ist in der Zeichnung dargestellt und wird nachfolgend im einzelnen beschrieben. Die einzige Figur zeigt in schematischer Darstellung die Stirnansicht eines Geräts zur Erzeugung von Kälte und Wärme, welches an ein Steuergerät angeschlossen ist.

Das als Ausführungsbeispiel gewählte Gerät weist einen Handgriff 1 auf. An dem Handgriff 1 ist ein Peltier-Element 2 befestigt. An der einen Seite des Peltier-Elements 2 liegt eine Platte 3 an, die bei Durchfließen eines Stroms kalt, an der anderen Seite eine Platte 4, die warm wird. Das Peltier-Element 2 und die Platten 3 und 4 sind von einer Isolierung 5 umschlossen. Auf die Platten 3 und 4 sind Behandlungsspitzen 11, 12 auf- und abschiebbar. Die Behandlungsspitzen 11, 12 sind in ihrer Ausgestaltung dem Anwendungsfall angepaßt, z.B. können sie an entgegengesetzten Ecken der Platten 3, 4 höckerartige Vorsprünge aufweisen, damit die Kalt- die Warmbehandlung nicht behindert und umgekehrt. Sie können auch die Gestalt von Behandlungflächen haben. Am Übergang zwischen den Platten 3, 4 und den Behandlungsspizen 11, 12 sind Temperaturfühler 6 vorgesehen.

Die für die Erzeugung der Wärme vorgesehene Platte 4 kann zusätzlich erwärmbar sein, zum Beispiel durch ein ebenfalls gesteuertes Heizelement 13. Auf der Seite der warmen Platte 4 ist in der Isolierung 5 ein Lüfter 14 vorgesehen.

Zur Steuerung des Peltier-Elements 2 und damit der an den Behandlungsspitzen 11, 12 der Platten 3, 4 herrschenden Temperatur ist das Gerät über seinen Handgriff 1 und Leitungen 7 mit einem Steuergerät 8 verbunden. Das Steuergerät 8 weist einmal Skalen 9 zur Anzeige der von den Temperaturfühlern 6 ermittelten Temperaturen im Bereich der Behandlungsspitzen 11, 12 auf. Zum anderen sind in dem Steuergerät 8 Stellglieder 10 vorgesehen, über die die Größe des fließenden Stromes und damit die Temperaturen eistellbar sind. Steuerungen für die zusätzliche Heizung 13 und für den Lüfter 14 sind ebenfalls in dem Steuergerät untergebracht. Mit den Stellgliedern 10 können nicht nur die gewünschten Temperaturen vorgewählt, sondern in Verbindung mit einem automatischen Regelkreis auch gehalten werden. Die Steuerung kann z.B. über einen Mikroprozessor erfolgen. Es ist zweckmäßig, in diesem Fall zusätzliche Skalen 9 vorzusehen, um sowohl die vorgewählten Temperaturen als auch die Ist-Temperatur im Bereich der Platten 3, 4/der Behandlungsspitzen 11, 12 anzuzeigen.

Das Gerät nach der Erfindung ist da einsetzbar, wo in schnellem Wechsel Kalt-Warm-Reize, z.B. in der Medizin, oder ein Temperaturgefälle, z.B. bei der Materialprüfung, erzeugt werden sollen.

## Patentansprüche

1. Elektronisch gesteuertes Gerät zur Erzeugung von Kälte und Wärme, bei dem an beiden Außenflächen eines von einem Strom steuerbarer Größe durchflossenen Peltier-Elements (2) Platten (3;4) aus leitendem Material anliegen, dadurch gekennzeichnet, daß jede Platte (3;4) mit einer auf- und abschiebbaren Behandlungsspitze (11;12) versehen und die an der warmen Seite des Peltier-Elements (2) anliegende Platte (4) zusätzlich beheizbar ist.

2. Gerät nach Anspruch 1, dadurch gekennzeichnet, daß das Peltier-Element (2) und die Platten (3;4) von einem mit einem Steuergerät (8) elektrisch verbundenen Handgriff (1) gehalten sind, an dem eine das Peltier-Element (2) und die Platten (3;4) bis auf deren Behandlungsspitzen (11;12) abdeckende Isolierung (5) befestigt ist.

3. Gerät nach Anspruch 2, dadurch gekennzeichnet, daß die Behandlungsspitzen (11;12) bezogen auf die Platten (3;4) auf- und abschiebbar sind.

4. Gerät nach einem der Ansprüche 1 bis 3, dadurch gekennzeichnet, daß auf der warmen Seite des Peltier-Elements (2) ein Lüfter (14) vorgesehen ist.

5. Gerät nach einem der Ansprüche 1 bis 4, dadurch gekennzeichnet, daß im Bereich der Behandlungsspitzen (11;12) an den Platten (3;4) Temperaturfühler (6) angebracht sind.

6. Gerät nach einem der Ansprüche 1 bis 5, dadurch gekennzeichnet, daß die Platten (3;4) aus Kupfer bestehen.

7. Gerät nach einem der Ansprüche 2 bis 6, dadurch gekennzeichnet, daß in dem Steuergerät (8) Stellglieder (10) vorgesehen sind, über die die Temperaturen an den Platten (3;4)/den Behandlungsspitzen (11;12) voreinstellbar und in Verbindung mit einem Regelkreis auf den eingestellten Temperaturen haltbar sind.

8. Gerät nach einem der Ansprüche 1 bis 7, dadurch gekennzeichnet, daß wenigstens zwei Peltier-Elemente so kaskadiert sind, daß an der warmen Seite des jeweils einen Elements die kalte Seite des jeweils anderen Elements direkt angrenzt.

## Claims

1. An electronically regulated apparatus for the generation of cold and heat, on which plates (3;4) are arranged on both outer faces of a Peltier element (2) through which a controllable current flows, characterised by the fact that each plate (3;4) is provided with an extendable and retractable treatment applicator (11;12) and in which the plate (4) located on the warm side of the Peltier element (2) can be additionally heated.

2. Apparatus as in Claim 1 characterised by the fact that the Peltier element (2) and the plates (3;4) are mounted on a handle (1) electrically connected to a control unit (8) and where the Peltier element (2) and the plates (3;4) are fitted with an insulating cover (5) extending up to their treatment applicators (11;12).

3. Apparatus as in Claim 1 characterised by the fact that the treatment applicators (11;12) can be extended and retracted relative to the plates (3;4).

4. Apparatus as in one of the Claims 1 to 3, characterised by the fact that a ventilator (14) is provided on the warm side of the Peltier element (2).

5. Apparatus as in one of the Claims 1 to 4, characterised by the fact that temperature probes (6) are arranged on the plates (3;4) in the area of the treatment applicators (11;12).

6. Apparatus as in one of the Claims 1 to 5, characterised by the fact that the plates (3;4) are made of copper.

7. Apparatus as in one of the Claims 2 to 6, characterised by the fact that regulating elements (10) are provided in the control unit (8) with which the temperatures on the plates (3;4) / the treatment applicators (11;12) can be preset and maintained at the selected temperatures by control circuitry.

8. Apparatus as in one of the Claims 1 to 7, characterised by the fact that at least two Peltier elements are cascaded such that the warm side of one element is contiguous with the cold side of the next element throughout.

## Revendications

1. Dispositif à commande électronique pour la génération du froid et de la chaleur dans lequel des plaques (3;4) en matériau conducteur s'appliquent contre les deux surfaces extérieures d'un élément Peltier traversé par un courant de grandeur réglable, caractérisé en ce que chaque plaque (3;4) est dotée d'une pointe (11;12) emmanchable et démanchable et que la plaque (4) en applique contre le côté chaud de l'élément Peltier (2) est en outre chauffable.

2. Dispositif selon revendication 1, caractérisé en ce que l'élément Peltier (2) et les plaques (3;4) sont retenus par une poignée (1) électriquement reliée à un appareil de commande (8), contre laquelle (1) est fixée une isolation (5) recouvrant l'élément Peltier (2) et les plaques (3;4) jusqu'aux pointes de traitement (11;12) de celles-ci (3;4).

3. Dispositif selon revendication 2, caractérisé en ce que les pointes de traitement (11;12) sont emmanchables et démanchables par rapport aux plaques (3;4).

4. Dispositif selon l'une des revendications 1 à 3, caractérisé en ce que du côté chaud de l'élément Peltier (2) est prévu un ventilateur (14).

5. Dispositif selon l'une des revendications 1 à 4, caractérisé en ce qu'au niveau des pointes de traitement (11;12), des capteurs thermométriques sont fixés contre les plaques (3;4).

6. Dispositif selon l'une des revendications 1 à 5, caractérisé en ce que les plaques (3;4) sont en cuivre.

7. Dispositif selon l'une des revendications 2 à 6, caractérisé en ce que des organes de réglage (10) sont prévus dans l'appareil de commande (8), par lesquels (10) on peut prérégler les températures régnant au niveau des plaques (3;4)/des pointes de traitement (11;12) et maintenir lesdites températures, en association avec un circuit régulateur, au niveau réglé.

8. Dispositif selon l'une des revendications 1 à 7, caractérisé en ce qu'au moins deux éléments Peltier sont montés en cascade de telle sorte qu'au côté chaud de chaque élément soit directement juxtaposé le côté froid de l'autre élément.
